# EUROPÄISCHE PATENTSCHRIFT

(11) **EP 4 496 528 B1**
(45) Veröffentlichungstag und Bekanntmachung des Hinweises auf die Patenterteilung: **13.05.2026**
(21) Anmeldenummer: 24720458.9
(22) Anmeldetag: 17.04.2024
(51) Int. Cl.: A61B 50/34, A61B 90/90

(54) **VORRICHTUNG ZUR POSITIONIERUNG EINES SIEBKORBS IN EINEM BEHÄLTER**
DEVICE FOR POSITIONING A SCREEN BASKET IN A CONTAINER
DISPOSITIF DE POSITIONNEMENT D'UN PANIER À TAMIS DANS UN RÉCIPIENT

(30) Priorität: 18.04.2023 DE 102023109767
(43) Veröffentlichungstag der Anmeldung: 29.01.2025
(73) Patentinhaber: Aesculap AG, 78532 Tuttlingen (DE)
(72) Erfinder: JANSEN-TROY, Arne, 78333 Stockach (DE); MAAS, Allan, 78467 Konstanz (DE); KIESSLING, Daniel, 78052 Villingen-Schwenningen (DE); HERMANN, Johannes, 72355 Schömberg (DE)
(74) Vertreter: DREISS Patentanwälte PartG mbB
(86) Internationale Anmeldenummer: PCT/EP2024/060339
(87) Internationale Veröffentlichungsnummer: WO 2024/218108

(56) Entgegenhaltungen:
- DE-A1- 102008 028 399
- DE-A1- 102009 022 185
- DE-A1- 102012 016 970
- DE-A1- 102020 103 131
- DE-A1- 102021 000 078

## Beschreibung

Die Erfindung geht aus von einem System, das eine Vorrichtung und einen Sterilgutbehälter umfasst.

In der DE 10 2020 131104 A1 ist eine Sterilisiersiebschale mit Fixiergriff offenbart, welche zum Einsetzen in und Entnehmen aus einer Containerwanne vorgesehen und ausgebildet ist.

DE 10 2009 022185 A1 offenbart ein Spanngitter. Ein elastisches Gitter ist so in einem Siebkorb eingespannt, dass ein Produkt, welches auf einem Siebkorbboden liegt, unter Anwendung einer Anpresskraft über das obere Gitter so fixiert oder gelagert wird, dass das Produkt durch die Bereiche mit erhöhter Verformbarkeit gehalten wird.

DE 10 2008 028399 A1 betrifft eine Vorrichtung zur Aufbereitung von medizinischen Instrumenten mit einer Schale und einem Deckel, der eine Einrichtung oder Ausgestaltung aufweist, welche der Festlegung von zumindest einer Einrichtung zum Fixieren der Instrumente dient.

DE 10 2020 103131 A1 betrifft ein Kennzeichnungssystem für medizintechnische Sterilcontainer und Siebschalen zur Aufnahme medizintechnischer Instrumente. Jedes Kennzeichnungsschild weist eine Schnittstelle auf. An jedem Sterilcontainer und jeder Siebschale ist eine zur Schnittstelle komplementäre Gegenschnittstelle ausgebildet. Die Schnittstelle und die Gegenschnittstelle sind zum haltenden Anordnen eines Kennzeichnungsschilds an einem der Sterilcontainer bzw. an einer der Siebschalen ausgebildet.

DE 10 2012 016970 A1 offenbart einen Siebkorb zur Aufnahme von medizinischen Instrumenten oder Geräten. Ein Siebkorbgebinde kann aus einem Sterilcontainer steril entnommen werden.

DE 10 2021 000078 A1 offenbart eine Siebkorbvorrichtung zur Aufbereitung von Medizinprodukten und für den Einsatz im OP.
- Die Erfindung ist in Anspruch 1 definiert.

Der Sterilgutbehälter wird nachfolgend kurz als "Behälter" bezeichnet.

Die in einer Aufsicht sichtbaren und maschinenlesbaren Schnittstellen sind insbesondere von außerhalb des Behälters sichtbar.

Die maschinenlesbare Schnittstelle zur Erkennung des Siebkorbs und/oder seiner Position und/oder seiner Orientierung ermöglicht ein verbessertes Greifen und Positionieren mittels eines Roboters.

In einer Ausführungsform weist die Vorrichtung eine mechanische Schnittstelle zum Greifen der Vorrichtung auf.

In einer Ausführungsform weist die Vorrichtung eine mechanische Schnittstelle zur Verbindung mit dem Siebkorb auf, insbesondere eine Nase und/oder einen Rastmechanismus zur kraft- und/oder formschlüssigen Verbindung mit dem Siebkorb, und/oder eine Fügestelle zum Verkleben und/oder Verschrauben mit dem Siebkorb. Diese Vorrichtung stellt ein Siebkorbpositionierelement dar welches an einem herkömmlichen Siebkorb ohne derartiges Siebkorbpositionierelement anordenbar ist. Die Siebkörbe selbst müssen nicht angepasst werden.

In einer Ausführungsform weist die Vorrichtung, insbesondere im Bereich der Nase und/oder des Rastmechanismus, wenigstens einen durch die Vorrichtung hindurchführenden Spülkanal auf. Dadurch eignet sich die Vorrichtung besonders für einen Einsatz in einem Behälter zur Sterilisation.

In einer Ausführungsform sind der Siebkorb und die Vorrichtung einstückig ausgebildet. Das bedeutet, der Siebkorb umfasst selbst die Funktion des Siebkorbpositionierelements.

Vorzugsweise umfasst die Schnittstelle zum Greifen die maschinenlesbare Schnittstelle zur Erkennung des Siebkorbs und/oder die maschinenlesbare Schnittstelle zur Erkennung der Position und/oder Orientierung des Siebkorbs. Diese Anordnung ist besonders kompakt.

Vorzugsweise umfasst die mechanische Schnittstelle zum Behälter eine Fläche zur Reduzierung eines Spiels zwischen Siebkorb und Behälter. Dadurch wird eine Abnutzung des Siebkorbs und des Behälters reduziert.

Vorzugsweise weist die Vorrichtung eine Fläche auf welche die maschinenlesbare Schnittstelle zur Erkennung des Siebkorbs und/oder die maschinenlesbare Schnittstelle zur Erkennung der Position des Siebkorbs umfasst.

In einer Ausführungsform ist die maschinenlesbare Schnittstelle zur Erkennung des Siebkorbs und/oder die maschinenlesbare Schnittstelle zur Erkennung der Position des Siebkorbs und/oder Orientierung in die Fläche eingeprägt oder auf die Fläche aufgedruckt. Diese Vorrichtung ist besonders einfach in Masse fertigbar.

In einer Ausführungsform ist die Fläche zum Stapeln eines weiteren Siebkorbs ausgebildet. Dadurch sind mehrere Siebkörbe übereinander anordenbar.

In einer Ausführungsform umfasst die mechanische Schnittstelle zum Greifen die Fläche. Dies ermöglicht ein besseres Greifen mit dem Roboter.

Vorzugsweise umfasst die maschinenlesbare Schnittstelle zur Erkennung des Siebkorbs einen optisch lesbareren Marker, insbesondere einen Barcode, einen Datamatrixcode, alphanummerische Daten oder einen QR Code, der eine Kennung des Siebkorbs maschinenlesbar codiert.

In einer Ausführungsform umfasst die maschinenlesbare Schnittstelle zur Erkennung der Position und/oder Orientierung ein Symbol mit einer eindeutigen Angabe einer Orientierung des Symbols. Dies ermöglicht eine unmissverständliche Anzeige der Orientierung.

Es kann vorgesehen sein, dass die Vorrichtung zumindest teilweise aus Kunststoff oder zumindest teilweise aus Metall gefertigt ist.

Es kann vorgesehen sein, dass die Vorrichtung einen Sender umfasst, der ausgebildet ist, ein elektromagnetisches Signal, insbesondere ein Bluetooth Low Energy Signal oder ein Radio Frequency Identification Signal, zur Erkennung des Siebkorbs zu senden, wobei die maschinenlesbare Schnittstelle zur Erkennung des Siebkorbs den Sender umfasst und/oder dass die Vorrichtung einen Sender umfasst, der ausgebildet ist, ein elektromagnetisches Signal, insbesondere ein Bluetooth Low Energy Signal oder ein Radio Frequency Identification Signal, zur Erkennung der Position und/oder Orientierung des Siebkorbs zu senden, wobei die maschinenlesbare Schnittstelle zur Erkennung der Position und/oder Orientierung den Sender umfasst.

Es kann vorgesehen sein, dass der Sender an die Vorrichtung geklebt ist oder dass die Vorrichtung zumindest teilweise aus Kunststoff gefertigt ist, wobei der Sender vom Kunststoff umgeben ist.

Die Erfindung betrifft insofern insbesondere eine als Siebkorbpositionierelement Vorrichtung, mit der ein Siebkorb nachgerüstet werden kann, eine solche Vorrichtung, die bereits im Auslieferungszustand an dem Siebkorb angebracht ist und eine solche Vorrichtung, die mit dem Siebkorb ausgebildet ist.

Weitere vorteilhafte Ausführungsformen sind der folgenden Beschreibung und der Zeichnung entnehmbar. In der Zeichnung zeigt:
- Figur 1A: eine perspektivische Darstellung eines Siebkorbpositionierelements,
- Figur 1B: eine Ansicht einer Unterseite des Siebkorbpositionierelements,
- Figur 1C: einen Ausschnitt aus der Ansicht,
- Figur 2A: eine Aufsicht auf einen Siebkorb mit zwei Siebkorbpositionierelementen in einem Sterilgutbehälter,
- Figur 2B: einen Ausschnitt aus der Aufsicht,
- Figur 3: eine perspektivische Darstellung von zwei gestapelten Siebkörbe mit jeweils zwei Siebkorbpositionierelementen,
- Figur 4: eine perspektivische Darstellung eines Siebkorbs mit zwei Siebkorbpositionierelementen eingesetzt in einen Sterilgutbehälter.

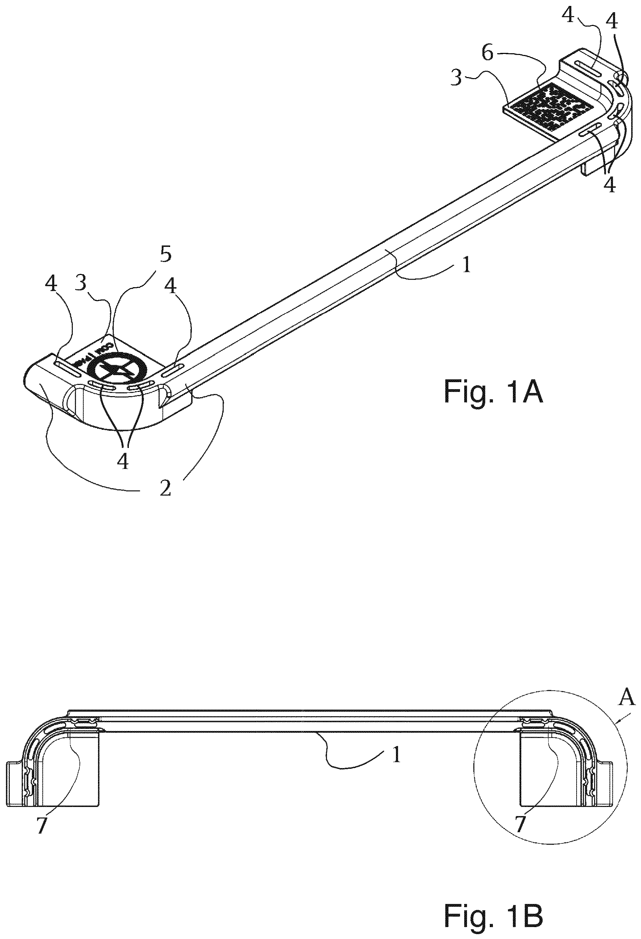

In Figur 1A ist ein Siebkorbpositionierelement 1 dargestellt. Das Siebkorbpositionierelement 1 ist ein Beispiel für eine Vorrichtung zur Positionierung eines Siebkorbs in einem Behälter. Der Behälter ist ein Sterilgutbehälter.

Das Siebkorbpositionierelement 1 ist am Siebkorb anordenbar. Das Siebkorbpositionierelement 1 ist im Beispiel an einem oberen Rand des Siebkorbs anordenbar.

Es kann vorgesehen sein, dass das Siebkorbpositionierelement stattdessen einstückig mit dem Siebkorb ausgeführt ist.

Das Siebkorbpositionierelement 1 kann in Kunststoff oder in Metall ausgeführt sein. Es kann vorgesehen sein, dass das Siebkorbpositionierelement 1 teilweise in Kunststoff und/oder teilweise in Metall ausgeführt ist.

Das Siebkorbpositionierelement 1 umfasst eine erste mechanische Schnittstelle 2 auf, welche zur Einschränkung einer Bewegung des Siebkorbs im Sterilgutbehälter ausgebildet ist. Die erste mechanische Schnittstelle 2 ist eine Schnittstelle zum Sterilgutbehälter. Die erste mechanische Schnittstelle 2 umfasst im Beispiel Flächen zur Reduzierung eines Spiels zwischen Siebkorb und Sterilgutbehälter. Die Flächen sind im Beispiel an wenigstens einem äußeren Randabschnitt des Siebkorbpositionierelementes 1 angeordnet. Im Beispiel sind drei Flächen vorgesehen. Es kann vorgesehen sein, dass die erste mechanische Schnittstelle 2 eine, zwei oder mehr als drei Flächen umfasst.

Das Siebkorbpositionierelement 1 umfasst eine zweite mechanische Schnittstelle 3 auf, die zum Greifen des Siebkorbpositionierelementes 1 ausgebildet ist. Die zweite mechanische Schnittstelle 3 ist eine Schnittstelle zum Greifen. Die zweite mechanische Schnittstelle 3 umfasst im Beispiel zwei Flächen. Es kann vorgesehen sein, dass die zweite mechanische Schnittstelle 3 eine oder mehr als zwei Flächen umfasst.

Das Siebkorbpositionierelement 1 weist im Beispiel zwei Eckstücke und einen Steg auf, der die zwei Eckstücke miteinander verbindet. Die Eckstücke sind im Beispiel als seitlicher Anschlag für einen Greifer zum Greifen des Siebkorbpositionierelementes 1 ausgebildet. Die Eckstücke umfassen im Beispiel je eine der zwei Flächen zum Greifen.

In einer Ausführungsform weist das Siebkorbpositionierelement 1 wenigstens einen Spülkanal 4 auf. Im Beispiel sind je Eckstück vier voneinander beabstandete Spülkanäle vorgesehen. Die Spülkanäle sind im Beispiel als Langloch ausgebildet. Die Spülkanäle können auch eine andere Form aufweisen. Es können auch mehr oder weniger als vier Spülkanäle vorgesehen sein.

In einer Ausführungsform umfasst das Siebkorbpositionierelement 1 eine erste maschinenlesbare Schnittstelle 5 zur Erkennung einer Position und/oder Orientierung des Siebkorbs. Die erste Schnittstelle 5 gibt die Position und/oder Orientierung des Siebkorbs an. Ein Koordinatensystem zur Navigation oder Positionsbestimmung ist z.B. bezüglich des Sterilgutbehälters ortsfest. Ausgehend von der erkannten Position der ersten maschinenlesbaren Schnittstelle 5 ist die Position des Siebkorbs bekannt. Ausgehend von der erkannten Orientierung der ersten maschinenlesbaren Schnittstelle 5 ist die Orientierung des Siebkorbs bekannt.

Die erste maschinenlesbare Schnittstelle 5 ist im Beispiel auf eine der Flächen für den Greifer aufgedruckt. Es kann auch vorgesehen sein, dass die erste maschinenlesbare Schnittstelle 5 in diese Fläche eingeprägt ist.

Es kann vorgesehen sein, dass die der ersten maschinenlesbare Schnittstelle 5 abgewandte Seite der Fläche einen Hinterschnitt oder mehrere Hinterschnitte umfasst. Dadurch wird ein sicheres Greifen oder Halten des Siebkorbes bei auftretenden Beschleunigungen durch Bewegungsrichtungswechsel ermöglicht.

Die erste maschinenlesbare Schnittstelle 5 umfasst in einer Ausführungsform ein Symbol mit einer eindeutigen Angabe einer Orientierung des Symbols. Im Beispiel umfasst das Symbol einen Kreis mit sich im rechten Winkel kreuzenden Diagonalen, die parallel zu je zwei der Seitenränder des Siebkorbs verlaufen, und in dem eine Kompassnadel in einem definierten Winkel zu den Diagonalen dargestellt ist. Es kann auch ein anderes Symbol vorgesehen sein. Beispielsweise umfasst das Symbol einen Pfeil, der parallel zu einer der Seitenränder oder in einem definierten Winkel zu den Seitenrändern verläuft. Ausgehend von der erkannten Orientierung der zweiten maschinenlesbaren Schnittstelle 5 ist die Orientierung des Siebkorbs bekannt.

Durch eine Positionsbestimmung ist damit die Position und/oder Orientierung des Siebkorbes relativ zum Sterilgutbehälter bestimmbar. Es kann vorgesehen sein, dass eine Höhe des Siebkorbes relativ zum Sterilgutbehälter nicht festgelegt ist.

Die erste maschinenlesbare Schnittstelle 5 umfasst in einer Ausführungsform einen ersten Sender. Der erste Sender ist ausgebildet, ein erstes elektromagnetisches Signal zur Erkennung einer Position und/oder Orientierung des Siebkorbs zu senden. Das erste elektromagnetische Signal ist z.B. ein Bluetooth Low Energy Signal oder ein Radio Frequency Identification Signal.

Es kann vorgesehen sein, dass die erste maschinenlesbare Schnittstelle 5 das Symbol und den ersten Sender umfasst.

In einer Ausführungsform umfasst das Siebkorbpositionierelement 1 eine zweite maschinenlesbare Schnittstelle 6 zur Erkennung des Siebkorbs.

Die zweite maschinenlesbare Schnittstelle 6 ist im Beispiel auf die andere der Flächen für den Greifer aufgedruckt. Es kann auch vorgesehen sein, dass die zweite maschinenlesbare Schnittstelle 6 in diese Fläche eingeprägt ist.

Es kann vorgesehen sein, dass die der zweite maschinenlesbare Schnittstelle 6 abgewandte Seite der Fläche einen Hinterschnitt oder mehrere Hinterschnitte umfasst. Dadurch wird ein sicheres Greifen oder Halten des Siebkorbes bei auftretenden Beschleunigungen durch Bewegungsrichtungswechsel ermöglicht.

Die zweite maschinenlesbare Schnittstelle 6 umfasst in einer Ausführungsform einen optisch lesbareren Marker, der eine Kennung des Siebkorbs maschinenlesbar codiert. Der optisch lesbare Marker ist z.B. ein Barcode oder ein Datamatrixcode oder ein QR Code. Der optisch lesbare Marker umfasst in einer Ausführungsform alphanummerische Daten.

Die zweite maschinenlesbare Schnittstelle 6 umfasst in einer Ausführungsform einen zweiten Sender. Der zweite Sender ist ausgebildet, ein zweites elektromagnetisches Signal zur Erkennung des Siebkorbs zu senden. Das zweite elektromagnetische Signal ist z.B. ein Bluetooth Low Energy Signal oder ein Radio Frequency Identification Signal.

Es kann vorgesehen sein, dass die erste maschinenlesbare Schnittstelle 5 und die zweite maschinenlesbare Schnittstelle 6 auf einer gemeinsamen Fläche angeordnet sind.

Es kann vorgesehen sein, dass statt dem ersten Sender und dem zweiten Sender ein gemeinsamer Sender vorgesehen ist, der das erste und das zweite elektromagnetische Signal sendet.

Der gemeinsame Sender oder der erste Sender oder der zweite Sender können an die Vorrichtung 1 geklebt sein. Es kann vorgesehen sein, dass die Vorrichtung 1 zumindest teilweise aus Kunststoff gefertigt ist und der gemeinsame Sender oder der erste Sender oder der zweite Sender vom Kunststoff umgeben ist.

In Figur 1B ist eine Ansicht einer Unterseite des Siebkorbpositionierelements 1 dargestellt. Das Siebkorbpositionierelement 1 weist eine dritte mechanische Schnittstelle 7 auf, die zur Verbindung des Siebkorbpositionierelements 1 mit dem Siebkorb ausgebildet ist. Die dritte mechanische Schnittstelle 7 ist eine Schnittstelle zum Siebkorb. Die dritte mechanische Schnittstelle 7 entfällt, wenn der Siebkorb und das Siebkorbpositionierelement 1 einstückig sind.

In diesem Fall der Einstückigkeit von Siebkorbpositionierelement 1 und Siebkorb 9 sind die beiden maschinenlesbaren Schnittstellen 5 und 6 auf der Oberseite von Laschen vorgesehen. Die Oberseite ist dabei entgegengesetzt zur in Figur 1B dargestellten Unterseite ausgerichtet. Die Laschen können in einem solchen Fall der Einstückigkeit aus demselben Blech bestehen wie der Siebkorb 9 bzw. eine Siebkorbwand. Zur Herstellung können diese Laschen dabei insbesondere aus demselben Blech ausgeschnitten sein wie der Siebkorb 9 bzw. die Siebkorbwand. Nach dem Ausschneiden der Laschen werden dieselben dann nach innen gebogen. Die Richtungsangabe "innen" bedeute dabei, dass die Laschen zumindest im Wesentlichen in einer Ebene liegend aufeinander weisend ausgerichtet sind. Die Angabe "aufeinander weisend" kann auch als "in Richtung auf eine Mündungsöffnung des Siebkorbs" aufgefasst werden. Diese Ebene liegt zumindest näherungsweise im Bereich der Mündungsöffnung des Siebkorbs 9.

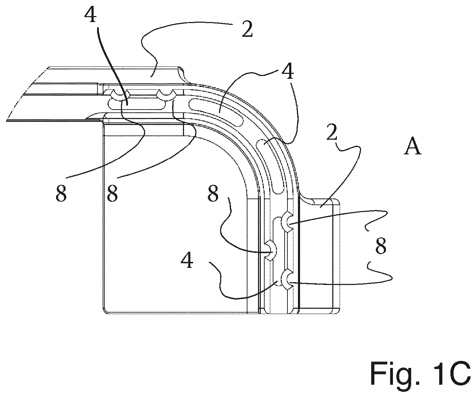

In Figur 1C ein Ausschnitt A aus der Ansicht dargestellt. Der Ausschnitt A umfasst eines der beiden Eckstücke. Im Beispiel sind die beiden Eckstücke gleich ausgebildet.

Im Beispiel umfasst die dritte mechanische Schnittstelle 7 je Eckstück sechs Nasen 8 zur kraft- und/oder formschlüssigen Verbindung mit dem Siebkorb. Es können mehr oder weniger als vier Nasen vorgesehen sein. Das Eckstück weist z.B. zwei Schenkel mit einer Nut auf. An einem Schenkel sind am Rand der Nut zwei Nasen in die Nut hineinragend angeordnet. An einem Schenkel sind am Rand der Nut zwei Nasen in die Nut hineinragend angeordnet, wobei zwischen den beiden Nasen eine Nase auf der gegenüberliegenden Seite der Nut in die Nut hineinragt. Die Nut ist zur Aufnahme des oberen Randes des Siebkorbs ausgebildet.

Im Bereich der Nasen ist im Beispiel je ein durch die Vorrichtung 1 hindurchführender Spülkanal 4 angeordnet.

Die dritte mechanische Schnittstelle 7 kann zudem oder stattdessen einen Rastmechanismus zur kraft- und/oder formschlüssigen Verbindung mit dem Siebkorb aufweisen.

Die dritte mechanische Schnittstelle 7 kann zudem oder stattdessen eine Fügestelle zum Verkleben aufweisen.

Die dritte mechanische Schnittstelle 7 kann zudem oder stattdessen eine Fügestelle zum Verschrauben mit dem Siebkorb aufweisen.

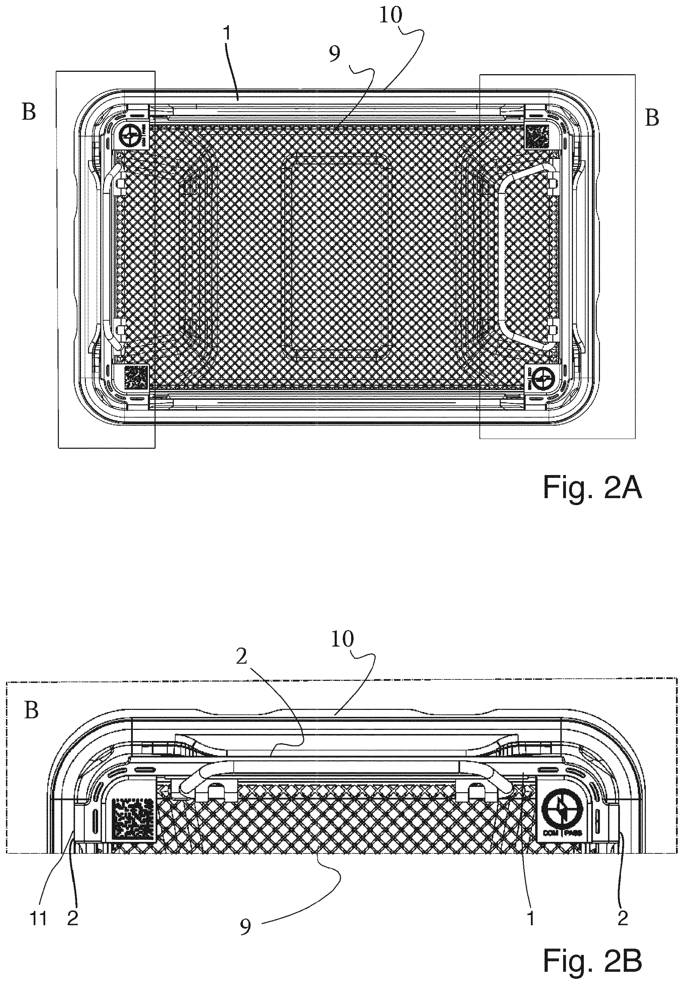

In Figur 2A ist eine Aufsicht auf einen exemplarischen Siebkorb 9 mit zwei Siebkorbpositionierelementen 1 in einem Sterilgutbehälter 10 dargestellt. Im Beispiel sind zwei gleiche Siebkorbpositionierelementen 1 vorgesehen. Die Siebkorbpositionierelemente 1 sind im Beispiel an einander gegenüberliegenden Seiten des Sterilgutbehälters 10 angeordnet.

Je ein Siebkorbpositionierelement 1 ist im Beispiel an einer kurzen Seite des Siebkorbs 9 angeordnet. Die Eckstücke sind jeweils an einer Ecke des Siebkorbs 9 angeordnet.

Die erste maschinenlesbare Schnittstelle 5 und die zweite maschinenlesbare Schnittstelle 6 sind in der Aufsicht auf den Sterilgutbehälter 10 von außerhalb des Sterilgutbehälters 10 sichtbar angeordnet.

In Figur 2B ist ein Ausschnitt B aus der Aufsicht dargestellt, der das Siebkorbpositionierelement 1 umfasst.

Die erste mechanische Schnittstelle 2 berührt im Beispiel eine innere Wand 11 einer Wanne des Sterilgutbehälters 10. Im Beispiel liegen die Flächen zur Reduzierung des Spiels zwischen Siebkorb 9 an der inneren Wand 11 des Sterilgutbehälters 10 an.

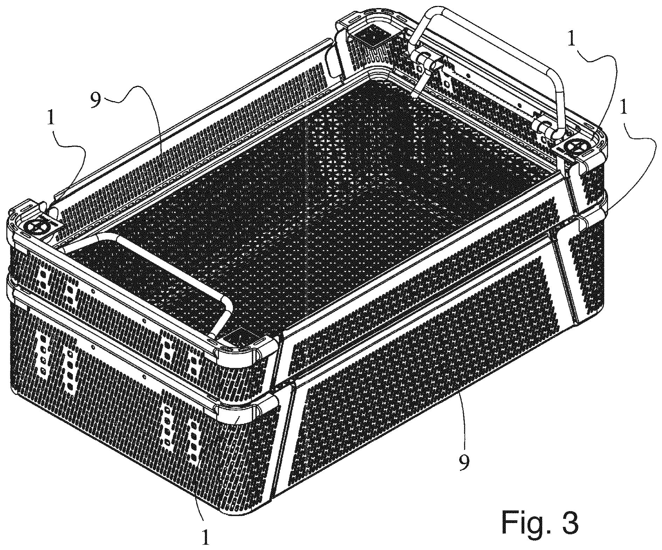

In Figur 3 ist eine perspektivische Darstellung von zwei gestapelten Siebkörben 9 mit jeweils zwei Siebkorbpositionierelementen 1 dargestellt. Die Siebkörbe 9 sind im Beispiel unterschiedlich hoch. Der obere Siebkorb 9 steht im Beispiel auf der am unteren Siebkorb 9 vorgesehenen Fläche zum Stapeln.

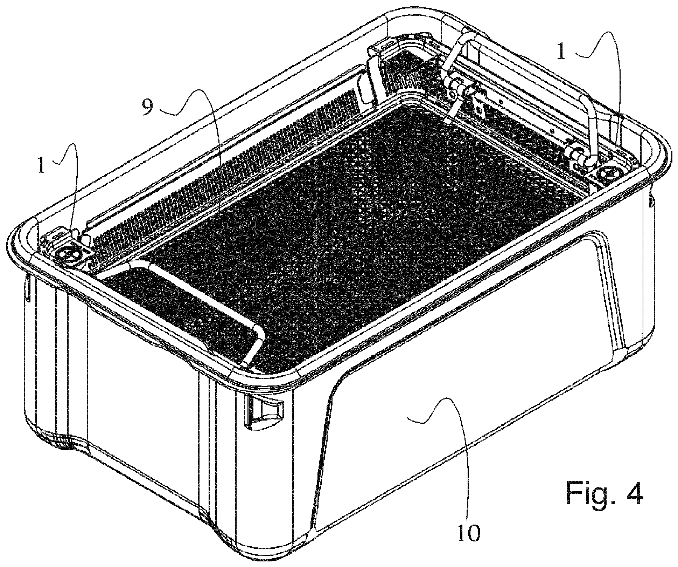

In Figur 4 ist eine perspektivische Darstellung des Siebkorbs 9 mit zwei Siebkorbpositionierelementen 1 dargestellt, der in den Sterilgutbehälter 10 eingesetzt ist.

In diesem System ist das Siebkorbpositionierelement 1 in die Wanne des Behälters 10 einsetzbar. Das Siebkorbpositionierelement 1 ist derart angepasst, dass die erste mechanische Schnittstelle 2 zur Einschränkung der Bewegung des Siebkorbs 9 im Behälter an einer Innenwand 11 der Wanne anliegt und den Siebkorb 9 gegen die Wanne verspannt und so im Behälter 10 lagefixiert.

## Patentansprüche

1. System, das eine Vorrichtung (1) und einen Sterilgutbehälter (10) umfasst, der eine Wanne zur Aufnahme eines Siebkorbs (9) umfasst, wobei die Vorrichtung (1) zur Positionierung des Siebkorbs (9) in dem Sterilgutbehälter (10) in die Wanne des Sterilgutbehälters (10) einsetzbar ist und derart angepasst ist, dass eine mechanische Schnittstelle (2) zur Einschränkung der Bewegung des Siebkorbs (9) im Sterilgutbehälter (10) an einer Innenwand (11) der Wanne anliegt und den Siebkorb (9) gegen die Wanne verspannt und so im Sterilgutbehälter (10) lagefixiert, , und wobei die Vorrichtung (1)
- eine in einer Aufsicht sichtbare und maschinenlesbare Schnittstelle (6) zur Erkennung des Siebkorbs (9) und/oder
- eine in einer Aufsicht sichtbare und maschinenlesbare Schnittstelle (5) zur Erkennung einer Position und/oder Orientierung des Siebkorbs (9), insbesondere in einem Koordinatensystem zur Navigation oder Positionsbestimmung,
aufweist.

2. System nach Anspruch 1, **dadurch gekennzeichnet, dass** die Vorrichtung (1) eine mechanische Schnittstelle (3) zum Greifen der Vorrichtung (1) aufweist.

3. System nach einem der vorherigen Ansprüche, **dadurch gekennzeichnet, dass** die Vorrichtung (1) eine mechanische Schnittstelle (7) zur Verbindung mit dem Siebkorb (9) aufweist, insbesondere eine Nase (8) und/oder einen Rastmechanismus zur kraft- und/oder formschlüssigen Verbindung mit dem Siebkorb (9), und/oder eine Fügestelle zum Verkleben und/oder Verschrauben mit dem Siebkorb (9).

4. System nach einem der vorherigen Ansprüche, **dadurch gekennzeichnet, dass** die Vorrichtung (1), insbesondere im Bereich der Nase (8) und/oder des Rastmechanismus, wenigstens einen durch die Vorrichtung (1) hindurchführenden Spülkanal (4) aufweist.

5. System nach einem der Ansprüche 1 oder 2, **dadurch gekennzeichnet, dass** der Siebkorb (9) und die Vorrichtung (1) einstückig ausgebildet sind.

6. System nach dem vorherigen Anspruch, **dadurch gekennzeichnet, dass** die beiden maschinenlesbaren Schnittstellen (5) und (6) auf Laschen vorgesehen sind, die einstückig mit dem Siebkorb (9) aus einem Blech geschnitten und nach innen gebogen sind.

7. System nach einem der vorherigen Ansprüche, **dadurch gekennzeichnet, dass** die Schnittstelle (3) zum Greifen die maschinenlesbare Schnittstelle (6) zur Erkennung des Siebkorbs (9) und/oder die maschinenlesbare Schnittstelle (5) zur Erkennung der Position und/oder Orientierung des Siebkorbs (9) umfasst.

8. System nach einem der vorherigen Ansprüche, **dadurch gekennzeichnet, dass** die mechanische Schnittstelle (2) zum Sterilgutbehälter eine Fläche zur Reduzierung eines Spiels zwischen Siebkorb (9) und Sterilgutbehälter umfasst.

9. System nach einem der vorherigen Ansprüche, **dadurch gekennzeichnet, dass** die Vorrichtung (1) eine Fläche aufweist welche die maschinenlesbare Schnittstelle (6) zur Erkennung des Siebkorbs (9) und/oder die maschinenlesbare Schnittstelle (5) zur Erkennung der Position und/oder Orientierung des Siebkorbs (9) umfasst.

10. System nach Anspruch 8, **dadurch gekennzeichnet, dass** die maschinenlesbare Schnittstelle (6) zur Erkennung des Siebkorbs (9) und/oder die maschinenlesbare Schnittstelle (5) zur Erkennung der Position und/oder Orientierung des Siebkorbs (9) in die Fläche eingeprägt ist, oder auf die Fläche aufgedruckt ist.

11. System nach einem der Ansprüche 8 oder 9, **dadurch gekennzeichnet, dass** die Fläche zum Stapeln eines weiteren Siebkorbs (9) ausgebildet ist.

12. System nach einem der Ansprüche 8 bis 10, **dadurch gekennzeichnet, dass** die mechanische Schnittstelle (3) zum Greifen die Fläche umfasst.

13. System nach einem der vorherigen Ansprüche, **dadurch gekennzeichnet, dass** die maschinenlesbare Schnittstelle (6) zur Erkennung des Siebkorbs (9) einen optisch lesbareren Marker, insbesondere einen Barcode, einen Datamatrixcode, alphanummerische Daten oder einen QR Code, umfasst, der eine Kennung des Siebkorbs (9) maschinenlesbar codiert.

14. System nach einem der vorherigen Ansprüche, **dadurch gekennzeichnet, dass** die maschinenlesbare Schnittstelle (5) zur Erkennung der Position und/oder Orientierung ein Symbol mit einer eindeutigen Angabe einer Orientierung des Symbols.

15. System nach einem der vorherigen Ansprüche, **dadurch gekennzeichnet, dass** die Vorrichtung (1) zumindest teilweise aus Kunststoff oder zumindest teilweise aus Metall gefertigt ist.

16. System nach einem der vorherigen Ansprüche, **dadurch gekennzeichnet, dass** die Vorrichtung (1) einen Sender umfasst, der ausgebildet ist, ein elektromagnetisches Signal, insbesondere ein Bluetooth Low Energy Signal oder ein Radio Frequency Identification Signal, zur Erkennung des Siebkorbs zu senden, wobei die maschinenlesbare Schnittstelle (6) zur Erkennung des Siebkorbs den Sender umfasst und/oder dass die Vorrichtung (1) einen Sender umfasst, der ausgebildet ist, ein elektromagnetisches Signal, insbesondere ein Bluetooth Low Energy Signal oder ein Radio Frequency Identification Signal, zur Erkennung der Position und/oder Orientierung des Siebkorbs (9) zu senden, wobei die maschinenlesbare Schnittstelle (5) zur Erkennung der Position und/oder Orientierung den Sender umfasst.

17. System nach Anspruch 16, **dadurch gekennzeichnet, dass** der Sender an die Vorrichtung (1) geklebt ist oder dass die Vorrichtung (1) zumindest teilweise aus Kunststoff gefertigt ist, wobei der Sender vom Kunststoff umgeben ist.

## Claims

1. System comprising a device (1) and a sterile-goods container (10) which comprises a trough for receiving a screen basket (9), wherein the device (1) is insertable into the trough of the sterile-goods container (10) in order to position the screen basket (9) in the sterile-goods container (10) and is adapted in such a way that a mechanical interface (2) for limiting the movement of the screen basket (9) in the sterile-goods container (10) rests against an inner wall (11) of the trough and braces the screen basket (9) against the trough, thus fixing the position of the screen basket in the sterile-goods container (10), and wherein the device (1) has
- a machine-readable interface (6), visible in a top view, for detecting the screen basket (9) and/or
- a machine-readable interface (5), visible in a top view, for detecting a position and/or orientation of the screen basket (9), in particular in a coordinate system for navigation or position determination.

2. System according to claim 1, **characterized in that** the device (1) has a mechanical interface (3) for gripping the device (1).

3. System according to either of the preceding claims, **characterized in that** the device (1) has a mechanical interface (7) for connection to the screen basket (9), in particular has a lug (8) and/or a locking mechanism for force-fitting and/or form-fitting connection to the screen basket (9), and/or a joint for adhesive bonding and/or screwing to the screen basket (9).

4. System according to any of the preceding claims, **characterized in that** the device (1), in particular in the region of the lug (8) and/or the locking mechanism, has at least one flushing channel (4) leading through the device (1).

5. System according to either of claims 1 and 2, **characterized in that** the screen basket (9) and the device (1) are formed in one piece.

6. System according to the preceding claim, **characterized in that** the two machine-readable interfaces (5) and (6) are provided on tabs which are cut from a metal sheet in one piece with the screen basket (9) and are bent inwards.

7. System according to any of the preceding claims, **characterized in that** the interface (3) for gripping comprises the machine-readable interface (6) for detecting the screen basket (9) and/or the machine-readable interface (5) for detecting the position and/or orientation of the screen basket (9).

8. System according to any of the preceding claims, **characterized in that** the mechanical interface (2) to the sterile-goods container comprises a surface for reducing play between the screen basket (9) and the sterile-goods container.

9. System according to any of the preceding claims, **characterized in that** the device (1) has a surface which comprises the machine-readable interface (6) for detecting the screen basket (9) and/or the machine-readable interface (5) for detecting the position and/or orientation of the screen basket (9).

10. System according to claim 8, **characterized in that** the machine-readable interface (6) for detecting the screen basket (9) and/or the machine-readable interface (5) for detecting the position and/or orientation of the screen basket (9) is embossed into the surface or is printed onto the surface.

11. System according to either of claims 8 and 9, **characterized in that** the surface is configured for stacking a further screen basket (9).

12. System according to any of claims 8 to 10, **characterized in that** the mechanical interface (3) for gripping comprises the surface.

13. System according to any of the preceding claims, **characterized in that** the machine-readable interface (6) for detecting the screen basket (9) comprises an optically readable marker, in particular a barcode, a data matrix code, alphanumeric data, or a QR code, which encodes an identifier of the screen basket (9) in a machine-readable manner.

14. System according to any of the preceding claims, **characterized in that** the machine-readable interface (5) for detecting the position and/or orientation a symbol with a unique indication of an orientation of the symbol.

15. System according to any of the preceding claims, **characterized in that** the device (1) is made at least partially of plastics or at least partially of metal.

16. System according to any of the preceding claims, **characterized in that** the device (1) comprises a transmitter which is configured to transmit an electromagnetic signal, in particular a Bluetooth low energy signal or a radio frequency identification signal, for detecting the screen basket, the machine-readable interface (6) for detecting the screen basket comprising the transmitter, and/or **in that** the device (1) comprises a transmitter which is configured to transmit an electromagnetic signal, in particular a Bluetooth low energy signal or a radio frequency identification signal, for detecting the position and/or orientation of the screen basket (9), the machine-readable interface (5) for detecting the position and/or orientation comprising the transmitter.

17. System according to claim 16, **characterized in that** the transmitter is adhesively bonded to the device (1) or **in that** the device (1) is made at least partially of plastics, the transmitter being surrounded by the plastics.

## Revendications

1. Système, comprenant un dispositif (1) et un récipient pour produits stériles (10) qui comprend une cuve pour la réception d'un panier à tamis (9), dans lequel le dispositif (1) peut être inséré dans la cuve du récipient pour produits stériles (10) pour positionner le panier à tamis (9) dans le récipient pour produits stériles (10) et est adapté de telle sorte qu'une interface mécanique (2) destinée à limiter le mouvement du panier à tamis (9) dans le récipient pour produits stériles (10) s'appuie contre une paroi intérieure (11) de la cuve et serre le panier à tamis (9) contre la cuve et le fixe ainsi en position dans le récipient pour produits stériles (10), et dans lequel le dispositif (1) présente
- une interface (6) visible en vue de dessus et lisible par machine pour la reconnaissance du panier à tamis (9) et/ou
- une interface (5) visible en vue de dessus et lisible par machine pour la reconnaissance d'une position et/ou d'une orientation du panier à tamis (9), en particulier dans un système de coordonnées pour la navigation ou la détermination de la position.

2. Système selon la revendication 1, **caractérisé en ce que** le dispositif (1) présente une interface mécanique (3) pour la préhension du dispositif (1).

3. Système selon l'une des revendications précédentes, **caractérisé en ce que** le dispositif (1) présente une interface mécanique (7) pour la liaison avec le panier à tamis (9), en particulier un ergot (8) et/ou un mécanisme d'encliquetage pour la liaison à force et/ou par complémentarité de forme avec le panier à tamis (9), et/ou un point d'assemblage pour le collage et/ou le vissage avec le panier à tamis (9).

4. Système selon l'une des revendications précédentes, **caractérisé en ce que** le dispositif (1) présente, en particulier dans la zone de l'ergot (8) et/ou du mécanisme d'encliquetage, au moins un canal de rinçage (4) traversant le dispositif (1).

5. Système selon l'une des revendications 1 ou 2, **caractérisé en ce que** le panier à tamis (9) et le dispositif (1) sont réalisés d'une seule pièce.

6. Système selon la revendication précédente, **caractérisé en ce que** les deux interfaces lisibles par machine (5) et (6) sont prévues sur des languettes qui sont découpées dans une tôle d'une seule pièce avec le panier à tamis (9) et sont pliées vers l'intérieur.

7. Système selon l'une des revendications précédentes, **caractérisé en ce que** l'interface (3) pour la préhension comprend l'interface lisible par machine (6) pour la reconnaissance du panier à tamis (9) et/ou l'interface lisible par machine (5) pour la reconnaissance de la position et/ou de l'orientation du panier à tamis (9).

8. Système selon l'une des revendications précédentes, **caractérisé en ce que** l'interface mécanique (2) avec le récipient pour produits stériles comprend une surface pour la réduction d'un jeu entre le panier à tamis (9) et le récipient pour produits stériles.

9. Système selon l'une des revendications précédentes, **caractérisé en ce que** le dispositif (1) présente une surface qui comprend l'interface lisible par machine (6) pour la reconnaissance du panier à tamis (9) et/ou l'interface lisible par machine (5) pour la reconnaissance de la position et/ou de l'orientation du panier à tamis (9).

10. Système selon la revendication 8, **caractérisé en ce que** l'interface lisible par machine (6) pour la reconnaissance du panier à tamis (9) et/ou l'interface lisible par machine (5) pour la reconnaissance de la position et/ou de l'orientation du panier à tamis (9) sont estampées dans la surface, ou sont imprimées sur la surface.

11. Système selon l'une des revendications 8 ou 9, **caractérisé en ce que** la surface est réalisée pour l'empilement d'un autre panier à tamis (9).

12. Système selon l'une des revendications 8 à 10, **caractérisé en ce que** l'interface mécanique (3) pour la préhension comprend la surface.

13. Système selon l'une des revendications précédentes, **caractérisé en ce que** l'interface lisible par machine (6) pour la reconnaissance du panier à tamis (9) comprend un marqueur plus lisible optiquement, en particulier un code à barres, un code matriciel de données, des données alphanumériques ou un code QR, qui codent de manière lisible par machine un identifiant du panier à tamis (9).

14. Système selon l'une des revendications précédentes, **caractérisé en ce que** l'interface lisible par machine (5) pour la reconnaissance de la position et/ou de l'orientation un symbole comportant une indication unique d'une orientation du symbole.

15. Système selon l'une des revendications précédentes, **caractérisé en ce que** le dispositif (1) est fabriqué au moins partiellement en matière plastique ou au moins partiellement en métal.

16. Système selon l'une des revendications précédentes, **caractérisé en ce que** le dispositif (1) comprend un émetteur qui est réalisé pour émettre un signal électromagnétique, en particulier un signal Bluetooth faible consommation ou un signal d'identification par radiofréquence, pour la reconnaissance du panier à tamis, dans lequel l'interface lisible par machine (6) pour la reconnaissance du panier à tamis comprend l'émetteur **et/ou en ce que** le dispositif (1) comprend un émetteur qui est réalisé pour émettre un signal électromagnétique, en particulier un signal Bluetooth faible consommation ou un signal d'identification par radiofréquence, pour la reconnaissance de la position et/ou de l'orientation du panier à tamis (9), dans lequel l'interface lisible par machine (5) comprend l'émetteur pour la reconnaissance de la position et/ou de l'orientation.

17. Système selon la revendication 16, **caractérisé en ce que** l'émetteur est collé sur le dispositif (1) ou **en ce que** le dispositif (1) est fabriqué au moins partiellement en matière plastique, dans lequel l'émetteur est entouré par la matière plastique.
